# EUROPEAN PATENT APPLICATION

(11) **EP 1 757 641 A1**
(43) Date of publication of application: **28.02.2007**
(21) Application number: 05018308.6
(22) Date of filing: 23.08.2005
(51) Int. Cl.: C08J 3/24, C08J 3/28, C08F 8/00, C08G 81/02, C08F 8/14, A61L 15/60, C08L 101/14

(54) **Method of surface cross-linking highly neutralized superabsorbent polymer particles using Bronsted acids**

(71) Applicant: The Procter and Gamble Company, Cincinnati, Ohio 45202 (US)
(72) Inventor: Meyer, Axel, 60486 Frankfurt (DE); Flohr, Andreas, 61476 Kronberg (DE); Lindner, Torsten, 61476 Kronberg (DE)
(74) Representative: Borbach, Markus

(57) **Abstract**

The present invention relates to a method of surface cross-linking superabsorbent polymer particles having a relatively high degree of neutralization. Brønsted acids are selectively applied onto the surface of the superabsorbent polymer particles to selectively facilitate a relatively high number of protonated carboxyl groups at the surface of the superabsorbent polymer particles while the relatively high degree of neutralization in the core of the superabsorbent polymer particles remains substantially unaffected.

## Description

### Field of the invention

The present invention relates to a method for making surface-cross-linked superabsorbent polymer (SAP) particles. The method uses SAP particles with a high degree of neutralization and further applies Brønsted acids. The present invention also relates to absorbent articles comprising SAP particles made by this method.

### Background of the invention

Superabsorbent polymers (SAPs) are well known in the art. They are commonly applied in absorbent articles, such as diapers, training pants, adult incontinence products and feminine care products to increase the absorbent capacity of such products while reducing their overall bulk. SAPs are capable of absorbing and retaining amounts of aqueous fluids equivalent to many times their own weight.

Commercial production of SAPs began in Japan in 1978. The early superabsorbent was a cross-linked starch-g-polyacrylate. Partially neutralized polyacrylic acid eventually replaced earlier superabsorbents in the commercial production of SAPs, and has become the primary polymer in SAPs.

SAPs are often applied in form of small particles. They generally consist of a partially neutralized lightly cross-linked polymer network, which is hydrophilic and permits swelling of the network once submerged in water or an aqueous solution such as physiological saline. The cross-links between the polymer chains assure that the SAP does not dissolve in water.

After absorption of an aqueous solution, swollen SAP particles become very soft and deform easily. Upon deformation the void spaces between the SAP particles are blocked, which drastically increases the flow resistance for liquids. This is generally referred to as "gel-blocking". In gel blocking situations liquid can move through the swollen SAP particles only by diffusion, which is much slower than flow in the interstices between the SAP particles.

One commonly applied way to reduce gel blocking is to make the particles stiffer, which enables the swollen SAP particles to retain their original shape thus creating or maintaining void spaces between the particles. A well-known method to increase the stiffness is to cross-link the acid groups (typically carboxyl groups) exposed in the surface of the SAP particles. This method is commonly referred to as surface cross-linking. Numerous different surface cross-linking molecules are known in the art, including (bifunctional) alcohols, carbonate diesters, epoxides, isocyanates, amines, and oxazolines. Surface cross-linking is commonly carried out at elevated temperatures of 150°C or above.

Commonly used surface cross-linking agents comprise diepoxy compounds, such as ethyleneglycol diglycidyl ether (available under the trade name Denacol from Nagase (Europa) GrnbH, Germany). The surface crosslinking reaction can be carried out at moderate temperatures (140°C).

A drawback of many surface cross-linking processes described above is that they require the presence of protonated acidic groups in order to achieve surface cross-linking at reasonable efficiency and/or reasonable speed. On the other hand, it is advantageous to use highly neutralized SAPs, as these typically can be manufactured at reduced cost compared to less neutralized SAPs. However, in neutralized SAPs the acidic groups are deprotonated and are in the form of the corresponding (mostly dissociated) salt.

Therefore, any neutralization of the SAP has to be carefully balanced with the need for surface cross-linking: The surface cross-linking agents known in the art only react at a sufficient speed with free acid groups comprised by the polymer chains but they are very slow / less efficient to react with neutralized acid groups. Thus, a given acid group can typically either be applied for surface cross-linking or for neutralization, but not for both. Surface cross-linking agents known in the art preferably react with acidic groups such as carboxylic acid or sulfonic acid groups, but they do not react with sufficient speed with neutralized acid groups such as carboxylates or sulfonates.

Therefore, SAPs known in the art are commonly only partially neutralized, e.g. to approximately 75 mol-% with sodium hydroxide.

An additional important aspect in the manufacturing of SAPs is the desire to reduce the amount of extractable polymer comprised by the SAPs (i.e. a polymer fraction that is soluble in excess liquid, and that is responsible for a decrease in SAP performance, especially by decreasing the capacity of the SAP particle).

The use of acids for the production and surface cross-linking of water-absorbent agents is also known in the art. However, so far the advantage of selectively using acids for surface cross-linking SAP particles having a high degree of neutralization has not been recognized. Also, the art typically teaches away from deliberate use of extractable polymer to improve surface cross-linking of SAP particles.

In the process of making SAP particles, neutralization of free carboxyl groups typically comes first, before surface cross-linking takes place. Indeed, the neutralization step is often carried out in the very beginning of the process, before the monomers are polymerized and cross-linked to form the SAP. Such a process is named 'pre-neutralization process'. Alternatively, the SAP can be neutralized during polymerization or after polymerization ('post-neutralization'). Furthermore, a combination of these alternatives is also possible.

It is therefore an objective of the present invention to provide a method of making SAP particles with homogenous surface cross-linking wherein SAP particles having a high degree of neutralization can be used.

It is a further objective of the present invention to provide an economic method of surface cross-linking SAP particles.

Alternatively to surface cross-linking methods comprised in the present invention, surface cross-linking can also be achieved by exposure to UV irradiation, as disclosed in the co-filed patent application titled "Method of surface cross-lmking superabsorbent polymer particles using ultraviolet radiation and Brønsted acids" (Attorney Docket # CM 3008FQ)

### Summary of the invention

The present invention relates to a method of surface cross-linking superabsorbent polymer particles. The method comprises the steps of:
a) providing superabsorbent polymer particles having a surface and a core;
b) applying one or more Brønsted acids onto the surface of said superabsorbent polymer particles; and
c) surface cross-linking the superabsorbent polymer particles;
wherein said superabsorbent polymer particles have a degree of neutralization of at least 80 mol-%.

Surface cross-linking according to the present invention is not achieved by exposing the superabsorbent polymer particles to UV radiation having a wavelength from 100 nm to 400 nm.

Optionally, one or more surface cross-cross-linking molecules can additionally be applied onto said surface of said superabsorbent polymer particles

### Detailed description of the invention

### Superabsorbent polymers

The SAPs according to the present invention preferably comprise a homo-polymer of highly neutralized α,β-unsaturated carboxylic acid or a copolymer of highly neutralized α,β-unsaturated carboxylic acid copolymerized with a monomer co-polymerizable therewith.

SAPs are available in a variety of chemical forms, including substituted and unsubstituted natural and synthetic polymers, such as carboxymethyl starch, carboxymethyl cellulose, and hydroxypropyl cellulose; nonionic types such as polyvinyl alcohol, and polyvinyl ethers; cationic types such as polyvinyl pyridine, polyvinyl morpholinione, and N, N-dimethylaminoethyl or N,N-diethylaminopropyl acrylates and methacrylates, and the respective quaternary salts thereof. Typically, SAPs useful herein have a multiplicity of anionic, functional groups, such as sulfonic acid, and more typically carboxyl groups. Examples of polymers suitable for use herein include those, which are prepared from polymerizable, unsaturated, acid-containing monomers. Thus, such monomers include the olefinically unsaturated acids and anhydrides that contain at least one carbon-to-carbon olefinic double bond. More specifically, these monomers can be selected from olefinically unsaturated carboxylic acids and acid anhydrides, olefinically unsaturated sulfonic acids, and mixtures thereof.

Some non-acid monomers can also be included, usually in minor amounts, in preparing SAPs. Such non-acid monomers can include, for example, the water-soluble or water-dispersible esters of the acid-containing monomers, as well as monomers that contain no carboxylic or sulfonic acid groups at all. Optional non-acid monomers can thus include monomers containing the following types of functional groups: carboxylic acid or sulfonic acid esters, hydroxyl groups, amide-groups, amino groups, nitrile groups, quaternary ammonium salt groups, aryl groups (e.g., phenyl groups, such as those derived from styrene monomer). These non-acid monomers are well-known materials and are described in greater detail, for example, in U.S. Patent 4,076,663 and in U.S. Patent 4,062,817.

Olefinically unsaturated carboxylic acid and carboxylic acid anhydride monomers include the acrylic acids typified by acrylic acid itself, methacrylic acid, ethacrylic acid, α-chloroacrylic acid, α--cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-sterylacrylic acid, itaconic acid, citroconic acid, mesaconic acid, glutaconic acid, aconitic acid, maleic acid, fumaric acid, tricarboxyethylene and maleic acid anhydride.

Olefinically unsaturated sulfonic acid monomers include aliphatic or aromatic vinyl sulfonic acids such as vinylsulfonic acid, allyl sulfonic acid, vinyl toluene sulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-methacryloxypropyl sulfonic acid and 2-acrylamide-2-methylpropane sulfonic acid.

Preferred SAPs according to the present invention contain carboxyl groups. These polymers comprise hydrolyzed starch-acrylonitrile graft copolymers, partially neutralized hydrolyzed starch-acrylonitrile graft copolymers, starch-acrylic acid graft copolymers, partially neutralized starch-acrylic acid graft copolymers, saponified vinyl acetate-acrylic ester copolymers, hydrolyzed acrylonitrile or acrylamide copolymers, slightly network cross-linked polymers of any of the foregoing copolymers, partially neutralized polyacrylic acid, and slightly network cross-linked polymers of partially neutralized polyacrylic acid, partially neutralized polymethacrylic acid, and slightly network cross-linked polymers of partially neutralized polymethacrylic acid. These polymers can be used either solely or in the form of a mixture of two or more different polymers, that when used as mixtures, individually do not have to be partially neutralized, whereas the resulting copolymer has to be. Examples of these polymer materials are disclosed in U.S. Patent 3,661,875, U.S. Patent 4,076,663, U.S. Patent 4,093,776, U.S. Patent 4,666,983, and U.S. Patent 4.734,478.

Most preferred polymer materials for use herein are slightly network cross-linked polymers of partially neutralized polyacrylic acids, slightly network cross-linked polymers of partially neutralized polymethacrylic acids, their copolymers and starch derivatives thereof. Most preferably, SAPs comprise partially neutralized, slightly network cross-linked, polyacrylic acid (i.e. poly (sodium acrylate/acrylic acid)). The SAPs for use in the present invention are at least 80 mol-% to 98 mol-%, more preferably at least 85 mol-% to 98 mol-% even more preferably at least 85 mol-% to 95 mol-% and even more preferably from 90 mol-% to 95 mol-% neutralized. Network cross-linking renders the polymer substantially water-insoluble and, in part, determines the absorptive capacity of the hydrogel-forming absorbent polymers. Processes for network cross-linking these polymers and typical network cross-linking agents are described in greater detail in U.S. Patent 4,076,663.

A suitable method for polymerizing α,β-unsaturated carboxylic acid monomers is aqueous solution polymerization, which is well known in the art. An aqueous solution comprising α,β-unsaturated carboxylic acid monomers and polymerization initiator is subjected to a polymerization reaction. The aqueous solution may also comprise further monomers, which are co-polymerizable with α,β-unsaturated carboxylic acid monomers. At least the α,β-unsaturated carboxylic acid has to be partially neutralized, either prior to polymerization of the monomers, during polymerization or post polymerization.

The monomers in aqueous solution are polymerized by standard free radical techniques, commonly by using a photoinitiator for activation, such as ultraviolet (UV) light activation. Alternatively, a redox initiator may be used. In this case, however, increased temperatures are necessary.

The water-absorbent resin will preferably be lightly cross-linked to render it water-insoluble. The desired cross-linked structure may be obtained by the copolymerization of the selected water-soluble monomer and a cross-linking agent possessing at least two polymerizable double bonds in the molecular unit. The cross-linking agent is present in an amount effective to cross-link the water-soluble polymer. The preferred amount of cross-linking agent is determined by the desired degree of absorption capacity and the desired strength to retain the absorbed fluid, that is, the desired absorption under load. Typically, the cross-linking agent is used in amounts ranging from 0.0005 to 5 parts by weight per 100 parts by weight of monomers (including α, β-unsaturated carboxylic acid monomers and possible comonomers) used. If an amount over 5 parts by weight of cross-linking agent per 100 parts is used, the resulting polymer has a too high cross-linking density and exhibits reduced absorption capacity and increased strength to retain the absorbed fluid. If the cross-linking agent is used in an amount less than 0.0005 parts by weight per 100 parts, the polymer has a too low cross-linking density and when contacted with the fluid to be absorbed becomes rather sticky, water-soluble and exhibits a low absorption performance, particularly under load. The cross-linking agent will typically be soluble in the aqueous solution.

Alternatively to co-polymerizing the cross-linking agent with the monomers, it is also possible to cross-link the polymer chains in a separate process step after polymerization.

After polymerization, cross-linking and partial neutralization, the wet SAPs are dehydrated (i.e. dried) to obtain dry SAPs. The dehydration step can be performed by heating the viscous SAPs to a temperature of about 120°C for about 1 or 2 hours in a forced-air oven or by heating the viscous SAPs overnight at a temperature of about 60°C. The content of residual water in the SAP after drying predominantly depends on drying time and temperature. According to the present invention, "dry SAP" refers to SAP with a residual water content of from 0.5% by weight of dry SAP up to 50% by weight of dry SAP, preferably, from 0.5% - 45% by weight of dry SAP, more preferably 0.5% - 30%, even more preferred 0.5% - 15% and most preferred 0.5% - 5%. If not explicitly said to be otherwise, in the following the term "SAP particles" refers to dry SAP particles.

The SAPs can be transferred into particles of numerous shapes. The term "particles" refers to granules, fibers, flakes, spheres, powders, platelets and other shapes and forms known to persons skilled in the art of SAPs. E.g. the particles can be in the form of granules or beads, having a particle size of about 10 µm to 1000 µm, preferably about 100 µm to 1000 µm. In another embodiment, the SAPs can be in the shape of fibers, i.e. elongated, acicular SAP particles. In those embodiments, the SAP fibers have a minor dimension (i.e. diameter of the fiber) of less than about 1mm, usually less than about 500 µm, and preferably less than 250 µm down to 50 µm. The length of the fibers is preferably about 3 mm to about 100 mm. Though less preferred for use in the present invention, the fibers can also be in the form of a long filament that can be woven.

The SAP particles of the present invention have a core and a surface. According to the present invention the dry SAP particles undergo a surface cross-linking process step, i.e. they are cross-linked in their surface while the number of cross-links in the core of the particle is not substantially increased by the method of the invention.

The term "surface" describes the outer-facing boundaries of the particle. For porous SAP particles, exposed internal surfaces may also belong to the surface. For the present invention, "surface" of the SAP particles refers to the complete and continuous outwardly facing 6 % volume of the dry SAP particle, whereas "core" refers to 94 % volume comprising the inner regions of the dry SAP particle.

The method of the present invention is used for surface cross-linking of SAP particles. Hence, the polymer chains comprised by the SAP particles already have been (core-) cross-linked by a cross-linker known in the art, comprising at least two polymerizable double bonds in the molecule unit.

The cross-linking of different polymer chains of the present invention is not intended to bond different SAP particles to each other. Thus, the method of the present invention does not lead to any appreciable inter-particulate bonds between different SAP particles but only results in intra-particulate direct covalent bonds within an SAP particle. If present, such inter-particulate direct covalent bonds would hence require additional inter-particulate cross-linking materials.

Surface cross-linked SAP particles are well known in the art. Surface cross-linking methods useful for the present invention are principally all surface cross-linking methods known in the art. In a surface cross-linked SAP particle the level of cross-links in the surface of the SAP particle is considerably higher than the level of cross-links in the core of the SAP particle.

### Surface cross-linking molecules

Typically, to achieve surface cross-linking a surface cross-linker is applied to the surface of the SAP particles. Commonly applied surface cross-linkers suitable for use in the present invention are thermally activatable surface cross-linkers. The term "thermally activatable surface cross-linkers" refers to surface cross-linkers, which only react upon exposure to increased temperatures, typically around 150 °C. Thermally activatable surface cross-linkers known in the prior art are e.g. di- or polyfunctional agents that are capable of building additional cross-links between the polymer chains of the SAPs. Typical thermally activatable surface cross-linkers include, e.g., di- or polyhydric alcohols, or derivatives thereof, wherein the derivatives are capable of forming di- or polyhydric alcohols. Representatives of surface cross-linking molecules are also alkylene carbonates, ketales, and di- or polyglycidylethers. Moreover, haloepoxy compounds, polyaldehydes, polyoles and polyamines are also well known thermally activatable surface crvss-linkers. The cross-linking is for example formed by an esterification reaction between a carboxyl group (comprised by the polymer) and a hydroxyl group (comprised by the surface cross-linker). As typically a relatively big part of the carboxyl groups of the polymer chain is neutralized prior to the polymerization step, commonly only few carboxyl groups are available for this surface cross-linking process known in the art. E.g. in a 70% percent neutralized polymer only 3 out of 10 carboxylic groups are available for covalent surface cross-linking.

Preferred surface cross-linking agents for use in the present invention are diepoxy compounds, such as ethyleneglycol diglycidyl ether (available under the trade name Denacol from Nagase (Europa) GmbH, Germany).

Further surface cross-linking agents for use in the method of the present invention are e.g. disclosed in column 11 of U.S. Patent 5,610,208 issued to Yorimichi et al on March 11, 1997.

Typically, surface cross-linking molecules are used for the method of the present invention. When surface cross-linking molecules are added to the SAP particles, additional covalent bonds are formed between the polymer chains comprised in the surface of the SAP particles. These additional covalent bonds comprise the reaction product of said surface cross-linking molecules with the acid groups of the SAP.

### Surface cross-linking without use of surface cross-linking molecules

Surface cross-linking according to the method of the present invention can however also be achieved without using any surface cross-linking molecules at all. E.g. surface cross-linking can be achieved by using e-beam.

Upon electromagnetic or electron beam irradiation, radicals can be formed in the polymer chains comprised in the surface of the SAP particles. Two such radicals comprised in different polymer chains comprised in the surface of the same SAP particle can combine to form a covalent bond between these two different polymer chains. Such radical formation may also be achieved via thermal or chemical generation of radicals.

Surface cross-linking of SAPs by means of e-beam processing can be performed using commercially-available accelerators, which are equipped with a variety of material handling systems, and are capable of significant throughput. A typical direct-current accelerator consists of the voltage generator, the electron gun, the accelerator tube, the scan hom, and the control system. This accelerator creates a beam of electrons approximately 2.5 centimeter in diameter and energizes it to near light speed. The beam passes through a scan hom, where a magnet scans it back and forth at ca. 200 Hz, creating a curtain of electrons 1-2 meters wide. Target materials are passed under the scan hom using conveyors, carts, reel-to-reel equipment, or other specialized handling means. For cross-linking of SAPs, accelerators with energies of 150 keV up to 5.0 MeV can be used.

With respect to processing economics, e-beam processing typically requires lower energy expenditure than conventional thermo-chemical processes to produce the same net effects.

### Neutralization

A major advantage of the present invention refers to the neutralization step: α,β-unsaturated carboxylic acid monomers are often neutralized prior to the polymerization step (pre-neutralization). Compounds, which are useful to neutralize the acid groups of the monomers are typically those, which will sufficiently neutralize the acid groups without having a detrimental effect on the polymerization process. Such compounds include alkali metal hydroxides, alkali metal carbonates and bicarbonates. Preferably, the material used for neutralization of the monomers is sodium- or potassium-hydroxide, or sodium- or potassium-carbonate. As a result, the carboxyl groups comprised by the α,β-unsaturated carboxylic acid of the polymer are at least partially neutralized. In case sodium hydroxide is used, neutralization results in sodium acrylate, which dissociates in water into negatively charged acrylate monomers and positively charged sodium ions. As the surface cross-linkers primarilyr react with the (carboxylic) acids comprised by the polymer and only react with the neutralized groups such as sodium acrylate, very slowly and ineffective, the degree of neutralization has to be balanced with the need to surface cross-link, because both process steps make use of the carboxyl groups.

If the final SAP particles are in the swollen state, after they absorbed aqueous solution, the sodium ions are freely movable within the SAP particles. In absorbent articles, such as diapers or training pants, the SAP particles typically absorb urine. Compared to distilled water, urine comprises a relatively high amount of salt, which at least partly is present in dissociated form. The dissociated salts comprised by the urine make absorption of liquid into the SAP particles more difficult, as the liquid has to be absorbed against an osmotic pressure caused by the ions of the dissociated salts. The freely movable sodium ions within the SAP particles strongly facilitate the absorption of liquid into the particles, because a higher degree of freely movable sodium ions within the SAP particles compared to the amount of freely movable sodium ions in the surrounding liquid increases the internal osmotic pressure. Therefore, a high degree of neutralization can increase the capacity of the SAP particles and the speed of liquid absorption.

Furthermore, a higher degree of neutralization typically reduces the materials expenses and, consequently, also reduces the overall manufacturing costs for SAP particles: Sodium hydroxide, which is commonly used to neutralize the polymer, is typically less expensive compared to acrylic acid, which is the most preferred polymer of today's SAPs. Hence, increasing the neutralization degree increases the amount of sodium hydroxide comprised by a given amount of SAPs. Consequently, less acrylic acid is required for making SAPs. Therefore, the method of the present invention provides an economically attractive way of making SAP particles.

### Brønsted acids

For surface cross-linking using SAP particles with a high degree of neutralization, Brønsted acids are able to considerably improve the surface cross-linking process as more surface cross-links can be formed in a given time interval.

In the method of the present invention, SAP particles with degrees of neutralization of from 80 mol-% to 98 mol-%, preferably from 85 mol-% to 98 mol-%, more preferably from 85 mol-% to 95 mol-% and most preferably from 90 mol-% to 95 mol-% are subjected to surface cross-linking.

The acid groups (typically the carboxylic acid groups (COOH)) comprised by the polymer of the SAP particles contribute to the overall reaction speed and efficiency of the surface cross-linking reaction.

However, for SAP particles having a relatively high degree of neutralization, most of the carboxyl groups are de-protonated (COO⁻), as they are in the form of the corresponding carboxylate salt (COOM with M being a monovalent metal cation such as Na⁺). It has now been found that this shortcoming of SAP particles with a relatively high degree of neutralization in light of surface cross-linking can be compensated by adding one or more Brønsted acids onto the surface of the SAP particles. It has further been found that thereby the overall concept of neutralization is not adversely affected. The Brønsted acid is capable of releasing protons (H⁺), thereby transferring the carboxylate salt in the surface of the SAP particle into the protonated form COOH.

By subjecting SAP particles with a high degree of neutralization of 80 mol-% or more to a treatment with one or more Brønsted acids, a low degree of neutralization can be selectively adjusted in the surface of the SAP particles, resulting in a more efficient reaction. At the same time, these SAP particles still have a relatively high degree of neutralization in the core of the SAP particles and hence, in the region making up the major part of the SAP particle. This is economically favorable due to the advantages of a high neutralization degree as described above.

Additionally to the Brønsted acid, a Lewis acid can be applied, preferably the aluminum cation Al³⁺, wherein Al³⁺ is preferably applied in the form of aluminum sulfate Al₂(SO₄)₃.

A Brønsted acid is any organic or inorganic compound capable of releasing protons (H⁺). Preferred Brønsted acids for the present invention are mineral acids like hydrochloric acid, sulphuric acid, phosphoric acid; saturated organic carbonylic acid like acetic acid, lactic acid, citric acid, succinic acid; oligomeric or polymeric organic acids like low molecular weight polyacrylic acid having a molecular weight MW of from 50 g/mol to 500 g/mol and saturated inorganic acids. A preferred saturated inorganic acid for use in the present invention is boric acid. The most preferred Brønsted acids according to the present invention are mineral acids and saturated organic carboxylic acids with the mineral acids being even more preferred than the carboxylic acids.

Another especially preferred group of Brønstedt acids for use in the present invention comprises polymeric acids, especially polyacrylic acids having a molecular weight (MW) (w) of from 700 g/mol to 5,000,000 g/mol. The use of these acids is especially preferred, as due to their high MW (w) and viscosity, they only penetrate slowly into the surface of the SAP particles. Use of polyacrylic acid also allows modifying the viscosity and surface tension of the surface cross-linking agents (if surface cross-linking molecules are used). Also, polyacrylic acid is relatively inexpensive, non toxic, not volatile at temperatures relevant for surface cross-linking methods known in the art and non corrosive.

The pKₐ value (dissociation index) of the Brønsted acid should be equal to or lower than the pKₐ value of the conjugated acid of the SAP repeat unit, which -in case of poly(meth)acrylic acid as polymer in the SAP particle- is typically between 4 and 5. Brønsted acids applied in the method of the present invention preferably have a pKₐ value of less than 5, more preferably less than 4 and most preferably less than 3. For example the Brønsted acid HCl, has a pKₐ value of -6.

However, apart from the pKa value, the effect of the acid on the particle flow behavior of the SAP particles during the irradiation may also influence the choice of the Brønsted acid. Some Brønsted acids may result in agglomeration of the SAP particles while others may even have a positive effect on the fluidity properties of the SAP particles (and may thus act as fluidity enhancers). The selection of the appropriate Brønsted acid therefore may have to be made depending on the given circumstances.

The amount of Brønsted acid applied in the method of the present invention is preferably in the range of from 0.005 weight-% to 10 weight-% by weight of SAP particles, more preferably from 0.01 weight-% to 5.0 weight-% and most preferably from 0.1 weight-% to 3.0 weight-% The amount of Brønsted acid also depends on the Brønsted acid which is used, and on the surface cross-linking molecules. The weight-ratio of Brønsted acid to surface cross-linking molecules ranges from 10:1 to 1:10, depending on the nature of the compounds.

In principle, also a mixture of several Brønsted acids can be used. However, this is less preferred as it increases the overall complexity of the method.

The Brønsted acid is preferably applied in water as an aqueous solution, as an emulsion or a suspension, before, preferably together with, or after the surface cross-linking molecules (if surface cross-linking molecules are used). A typical concentration of the Brønsted acid in an aqueous solution is 1 mol/l to 2 mol/l (with respect to Brønsted acidic protons) Alternatively, the Brønsted acid can also be applied separately from the surface cross-linking molecules (if surface cross-linking molecules are used).

Also, the Brønsted acids can be applied while dissolved or suspended in alcohol, e.g. isopropanol. The advantage of using alcohol instead of water is that alcohol does not migrate into the SAP particles to a substantial degree. Hence, it is easier to control the penetration depth in order to avoid Brønsted acids migrating onto the core. Thereby it is easier to ensure that the surface cross-linking reaction is actually restricted to the surface of the SAP particles. The alcohol may be removed (via evaporation) prior to surface cross-linking of the SAP particles.

If the Brønsted acids are applied in a mixture of alcohol and water, the penetration depth of the mixture -and thereby of the Brønsted acids- can be carefully adjusted by choosing the appropriate ratio between alcohol and water.

It may also be desirable to apply the Brønsted acid suspended in water, choosing a Brønsted acid which does not dissolve in water very well. Thereby it is also possible to ensure that the Brønsted acids actually remain in the surface of the SAP particles and do not migrate into the core together with the water.

Also, use of polymeric acids as Brønsted acid also helps to restrict surface cross-linking to the surface of the SAP particles as polymeric acid molecules are typically to big to penetrate substantially into the core of the SAP particles. Polymeric acids as Brønsted acid further enables surface cross-linking wherein the surface cross-links are substantially uniformly distributed in the surface of the SAP particles as the polymeric acid becomes incorporated into the overall surface cross-linking structure.

Generally, the reaction partners should be mixed well before surface cross-linking to improve yield of the surface cross-linking reaction, resulting in reduced levels of residual surface cross-linking molecules.

The Bronsted acid can be applied onto the SAP particles prior to applying the surface cross-linking molecules (if surface cross-linking molecules are used). If the Brønsted acid is applied in water, it is preferred that it is applied immediately before the surface cross-linking reaction takes place to ensure that the Brønsted acid does not migrate into the core to a substantial degree. Preferably, the Brønsted acid should not be applied more than 10 min prior to starting the surface cross-linking reaction, more preferably not more than 5 minutes and most preferably the time between application of the Brønsted acid and start of the surface cross-linking should not be more than 1 minute, especially if the Brønsted acid is applied in water.

Fluidity enhancers, as they are widely known in the art, such as hydrophilic amorphous silicas, as they are commercially available e.g. from Degussa Corp., can optionally be added to the SAP particles to assist in avoiding agglomerates, e.g if the water content of the SAP particles is relatively high. The fluidity enhancers are typically applied in a range of from 0.1 weight-% by weight of SAP particles to 10 weight-% by weight of SAP particles.

For applying the Brønsted acids and (if used) the surface cross-linking molecules and / or for surface cross-linking the SAP particles according to the present invention, a fluidized bed reactor having a radial symmetric geometry or vibrating plates may be used.

However, for the method of the present invention, it should be ensured that the Brønsted acids and (if applicable) the surface cross-linking molecules are homogeneously applied onto the SAP particles.

### Absorbent articles

The SAP particles made by the method of the present invention are preferably applied in absorbent cores of absorbent articles. As used herein, absorbent article refers to devices that absorb and contain liquid, and more specifically, refers to devices that are placed against or in proximity to the body of the wearer to absorb and contain the various exudates discharged from the body. Absorbent articles include but are not limited to diapers, adult incontinent briefs, diaper holders and liners, sanitary napkins and the like.

Preferred absorbent articles of the present invention are diapers. As used herein, "diaper" refers to an absorbent article generally worn by infants and incontinent persons about the lower torso.

Absorbent articles especially suitable for the present invention typically comprise an outer covering including a liquid pervious topsheet, a liquid impervious backsheet and an absorbent core generally disposed between the topsheet and the backsheet. The absorbent core may comprise any absorbent material that is generally compressible, conformable, non-irritating to the wearer's skin, and capable of absorbing and retaining liquids such as urine and other certain body exudates. In addition to the SAP particles of the present invention, the absorbent core may comprise a wide variety of liquid-absorbent materials commonly used in disposable diapers and other absorbent articles such as comminuted wood pulp, which is generally referred to as air felt.

Exemplary absorbent structures for use as the absorbent assemblies are described in U.S. Patent No. 5,137,537 entitled "Absorbent Structure Containing Individualized, Polycarboxylic Acid Crosslinked Wood Pulp Cellulose Fibers" which issued to Herron et al. on August 11, 1992; U.S. Patent 5,147,345 entitled "High Efficiency Absorbent Articles For Incontinence Management" issued to Young et al. on September 15, 1992; U.S. Patent No. 5,342,338 entitled "Disposable Absorbent Article For Low-Viscosity Fecal Material" issued to Roe on August 30, 1994; U.S. Patent No. 5,260,345 entitled "Absorbent Foam Materials For Aqueous Body Fluids and Absorbent Articles Containing Such Materials" issued to DesMarais et al. on November 9, 1993; U.S. Patent No. 5,387,207 entitled "Thin-Until-Wet Absorbent Foam Materials For Aqueous Body Fluids And Process For Making Same" issued to Dyer et al. on February 7, 1995; U.S. Pat. No. 5,397,316 entitled "Slitted Absorbent Members For Aqueous Body Fluids Formed Of Expandable Absorbent Materials" issued to LaVon et al on March 14, 1995; and U.S. Patent No. 5,625,222 entitled "Absorbent Foam Materials For Aqueous Fluids Made From high In al. on July 22, 1997.

All documents cited in the Detailed Description of the Invention, are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

Each dimension for which a value is defined herein is a technical dimension, which in the context of the present invention is not to be understood literal. Hence, all embodiments having dimensions functionally equivalent to the dimensions stated herein are intended to be covered by the scope of the invention, e.g. a dimension of "40 mm" has to be understood as meaning "about 40 mm".

## Claims

1. A method of surface cross-linking superabsorbent polymer particles which comprises the steps of:
a) providing superabsorbent polymer particles having a surface and a core;
b) applying one or more Brønsted acids onto said surface of said superabsorbent polymer particles; and
c) surface cross-linking said superabsorbent polymer particles, said surface cross-linking not being achieved by exposing said superabsorbent polymer particles to UV radiation having a wavelength from 100 nm to 400 nm;
wherein said superabsorbent polymer particles have a degree of neutralization of at least 80 mol%.

2. The method according to claim 1, wherein additionally one or more surface cross-linking molecules are applied onto said surface of said superabsorbent polymer particles.

3. The method according to claim 2, wherein said surface cross-linking molecules are-thermally activatable surface cross-linking molecules and wherein said surface cross-linking is achieved by exposing said superabsorbent polymer particles with said Brønsted acids and said surface cross-linking molecules applied on said surface to a temperature of at least 80°C, preferably at least 110°C.

4. The method according to claim 3, wherein said thermally activatable surface cross-linking molecules are di- or polyhydric alcohols, or derivatives.

5. The method according to claim 3, wherein said thermally activatable surface cross-linking molecules are diepoxy compounds, such as ethyleneglycol diglycidyl ether.

6. The method according to claim 1, wherein said surface cross-linking is achieved by exposing said superabsorbent polymer particles with said Brønsted acids applied on said surface to electromagnetic or electron beam irradiation.

7. The method according to any of the preceding claims, wherein said Brønsted acids are mineral acids or saturated organic carboxylic acids.

8. The method according to any of claims 1 to 6, wherein said Brønsted acids are polymeric organic acids.

9. The method according to claim 8, wherein said Brønsted acids are polyacrylic acid.

10. Absorbent article comprising superabsorbent polymer particles made according to the method of any of the preceding claims.
